Europäisches Patentamt

European Patent Office

Office européen des brevets

(19)

(11) Publication number: 0 399 119
A1

(12) **EUROPEAN PATENT APPLICATION**

(21) Application number: 89307677.8

(22) Date of filing: 27.07.89

(51) Int. Cl.5: **A61M 5/142**

(30) Priority: 25.05.89 US 357040

(43) Date of publication of application:
28.11.90 Bulletin 90/48

(84) Designated Contracting States:
**DE FR GB**

(71) Applicant: **IMED CORPORATION**
**9925 Carroll Canyon Road**
**San Diego California 92131(US)**

(72) Inventor: **Hyman, Oscar, E.**
**1522 Calle Tulipanes**
**Encinitas California 92024(US)**
Inventor: **Aten, Michael R.**
**13720 Via Huelva**
**San Diego California 92121(US)**

(74) Representative: **Coxon, Philip et al**
**Eric Potter & Clarkson St. Mary's Court St.**
**Mary's Gate**
**Nottingham NG1 1LE(GB)**

(54) **An intravenous fluid infusing device.**

(57) An ambulatory programmable pump (10) for infusing fluid to a patient having a built in display of user instructions comprises a foldable casing (12) having pumping apparatus (36) mounted therein and including fluid container means (30) for storing fluid to be infused to a patient. A central processing unit (50) is carried in the casing and connected to the pumping means, which is controlled by a keyboard (18) having keys (26) mounted on the casing for controlling operation of the processor and pumping means. A folding display (20) is hingedly connected to the casing and is movable between an open position for displaying instructions and information to the user to allow operation of the device, and a closed position where the display is protected from view. Also included are status indicating lights (102,104,106) and an audible alarm (108). The display screen has large, easily readable lettering, and the central processing unit generates prompting stimulus on the display for providing the user with instructions and requesting information to be entered into the keyboard, including rate of infusion and rate of infusion and volume of fluid to be infused. Also included is a method for carrying out same.

FIG.1

## AN INTRAVENOUS FLUID INFUSING DEVICE

This invention relates generally to intravenous (I.V.) fluid infusing devices and to methods of operating fluid transferring devices. More particularly, this invention relates to portable I.V. fluid infusion devices which are easily adapted for use by the ambulatory patient in a home or other non-medical environment. In particular, this invention relates to ambulatory programmable pumps and to methods of operating said pumps. This invention is particularly, but not exclusively, useful for providing I.V. fluids to a patient which include therapeutic drugs, analgesics and the like.

Over the years, there have been many devices for infusing medicinal fluids to patients in the process of receiving medical services and therapies. Such apparatus and processes, however, have typically been utilized in a hospital or other medical care environment. In addition, such devices are required to be operated by nurses, physicians, or other health care professionals who are trained in the operation and use of such fluid infusion devices. This is required, in part, as a result of the need for proper placement of needles into the patient and the like for introduction of the fluids, and further for establishing proper rates of flow and volumes of medicinal fluids to the patient.

Fairly recently, it has been recognized that there are a number of I.V. therapies and other parental administration of therapeutic medicaments which can be administered in a setting other than the hospital or other medical environment. Such settings include the home setting. Such devices are typically portable devices for infusion of drugs and other medicaments. Typical devices include intermittent syringe pumps, continuous peristaltic pumps and elastomeric or balloon powered drug reservoirs. The patient may often have a surgically implanted valve for allowing attachment to the I.V. infusion device. In addition, microprocessor controlled, portable infusion pumps are available for varying flow rates, and also volume capacity adjustments. Such devices are useful for cancer chemotherapy, delivery of analgesia and other pain killing medicaments to a patient in a continuous flow fashion, and for anticoagulation therapy. In addition, therapy for such conditions as diabetes and nutritional support, as well as treatment of infectious diseases such as osteomyelitis find such mobile devices useful. Ambulatory drug delivery mechanism are also useful for hormonal therapy utilizing fertility drugs and such things as pituitary failure in adolescents. Such mobile devices allow treatment to take place outside of the hospital environment since the devices are portable and can be worn by the ambulatory uses at all times.

In such conventional portable devices, however, there is much difficulty for someone other than a nurse or other trained medical professional to utilize such devices, because their operation is extremely complicated. Typically, extensive training is required to allow operation of some of these complex ambulatory portable infusion devices which utilize many controls and steps in order to set up and operate the devices. Many times a special training course in programming the device may be required for the operator of the device. Out of necessity, nurses are typically utilized to assist the patient in assuring proper settings for continuous ambulatory fluid infusion. As a consequence, the ability to use such systems in a home setting is significantly limited by the number of trained technicians available to set up and enable operation of such complicated portable devices.

It is an object of the present invention to provide an improved intravenous fluid infusing device. It is another object of the present invention to provide an improved method of operating a fluid transferring device.

According to one aspect of this invention there is provided an intravenous fluid infusing device comprising:
a foldable casing;
computer controlled pumping means mounted in said casing for pumping fluid;
a keyboard mounted on said casing for controlling operation of said pumping means: and
display means mounted in said casing and hingedly attached to said keyboard for displaying information in response to input to said keyboard.

According to another aspect of this invention there is provided an ambulatory programmable pump having a built in display of user instructions, comprising:
a body portion including a processor, memory, keyboard and peristaltic pumping apparatus operatively connected for pumping fluid to a patient; and
a cover portion hingedly attached to said body portion, said cover portion having an inside surface carrying a display screen connected to said processor for indicating prompting stimulus to the user in response to user input to said keyboard.

According to another aspect of this invention, there is provided an ambulatory programmable pump for infusing fluid to a patient, comprising:
a foldable casing having pumping means mounted therein and including fluid container means for storing fluid to be infused to a patient;
a central processing unit carried in said casing and connected to said pumping means;

a keyboard having keys mounted on said casing for controlling operation of said processor and said pumping means; and

foldable display means hingedly connected to said casing moveable between an open position for displaying information to the operator of the pump, said information including step-by step instructions to operate the pump, and a closed position wherein said display is protected from view.

According to another aspect of this invention there is provided an ambulatory programmable pump, comprising:

a foldable casing including a computer controlled pumping means for pumping I.V. fluid to a patient at desired rates and in desirable amounts;

said foldable casing having a body portion having a keyboard mounted thereon for controlling operation of said pumping means; and

said foldable casing further having a cover portion hingedly attached to said body portion, said cover portion having an inside surface and an outside surface, the inside surface of said cover portion having a display screen mounted thereon for displaying information for instructing the user in operation of the dispenser.

According to another aspect of this invention there is provided a method of operating a fluid transferring device having a peristaltic pumping apparatus, comprising the steps of:

attaching a fluid reservoir in operative engagement to said peristaltic pumping apparatus;

opening a cover carrying a foldable display screen for viewing user instructions generated by said computerized pump;

entering data including transfer rate and volume of said fluid via said keyboard in response to prompting stimulus displayed to the user on said foldable display screen; and

closing said cover and enabling said device to transfer said fluid at the rate and in the amount entered by said user.

The present invention thus recognizes the need for an ambulatory programmable pump device which is convenient and easy to use. The present invention also recognizes the need for a device which can be utilized in a home environment by unskilled people, whether the user be old, young, sick, healthy, family, or friends. A requirement for specialized training should not be necessary. A user should preferably be able to easily set up and operate such a system with the desired flow settings and amounts in order to truly realize a convenient and useful device. It is desirable that there be a device which enables a user to connect the device to a fluid source, conveniently set the operating parameters, and put the device into operation, without special instructions, specialized training, or professional assistance.

In light of the above, it is an advantage of the present invention that it provides a portable device for infusing fluid to a patient which is simple and efficient to use. Another advantage of the present invention is that it provides an ambulatory programmable pump device which requires no specialized training in order to operate the device. Yet another advantage of the present invention is that it provides an ambulatory programmable pump device which is relatively easy to manufacture and which is cost effective.

A preferred embodiment of the ambulatory programmable pump comprises a foldable casing for housing a computer and an I.V. fluid pump, as well as a fluid container for storing I.V. fluid to be infused to a patient. In the preferred embodiment a keyboard is mounted on a body portion of the casing for input of information to the computer for controlling operation of the pump. In this embodiment a folding display is hingedly connected to the body of the casing, movable between an open position for displaying the information to the operator of the device including step-by-step instructions, and a closed position where the display is protected from view.

The folding display may be a liquid crystal display (LCD) type display. The computer may generate a step-by-step instruction format on the display for communicating necessary information to the user concerning the status of the device and requesting the information required to enable its operation. For example, in a normal mode, the operator is requested to enter the rate of infusion via the keyboard. As each number is entered, it is displayed on the screen, and when the proper rate is fixed by pressing an enter key, the data is stored in the computer. The user need simply read and follow instructions as they are displayed, step-by-step, on the fold out display. Thus, no specialized training is required. Information requested via the display includes, for example, rate of infusion in milliliters per hour, volume of fluid to be infused in milliliters, and similar information. In the program mode, the user enters the rate, the total time to infuse a specified volume, the elapsed off-time and the elapsed restart time. In a patient controlled analgesic mode, the user is instructed to enter a basic low level rate of infusion, and to enter an amount of extra bolus of analgesic fluid to be infused to the patent on command. When the display is not in use, the casing can be folded closed so that the display is protected, and the device is conveniently shaped to be easily carried on the body of the ambulatory patient.

Reference is now made to the accompanying drawings, in which:

Figure 1 is a perspective view of a portable ambulatory programmable pump according to the present invention;

Figure 3 is a schematic diagram showing a preferred arrangement of component parts of an ambulatory programmable pump in accordance with the present invention;

Figure 4 shows a flow diagram of the sequence of prompting stimuli in accordance with the present invention:

Figure 5 shows a continuation of the flow diagram of the sequence of prompting stimuli depicted in Figure 4; and

Figure 6 shows a detail flow diagram of prompting stimuli depicted as "status indicator/alarm functions" in Figure 5.

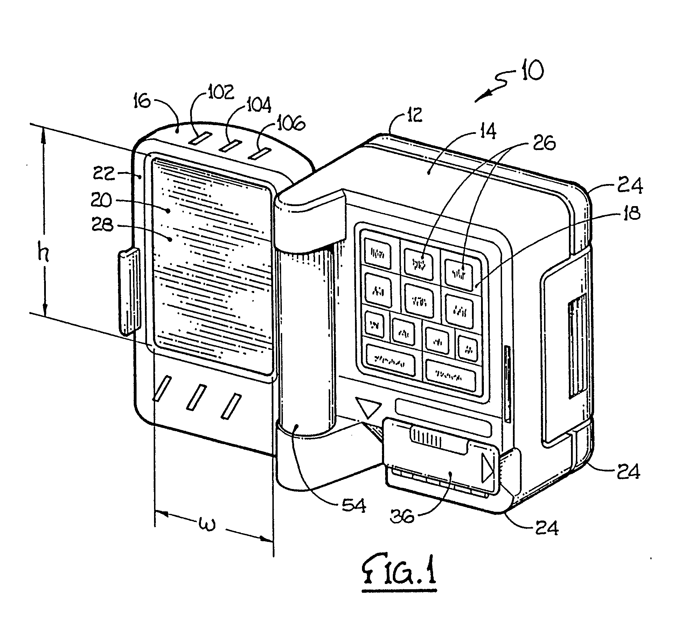

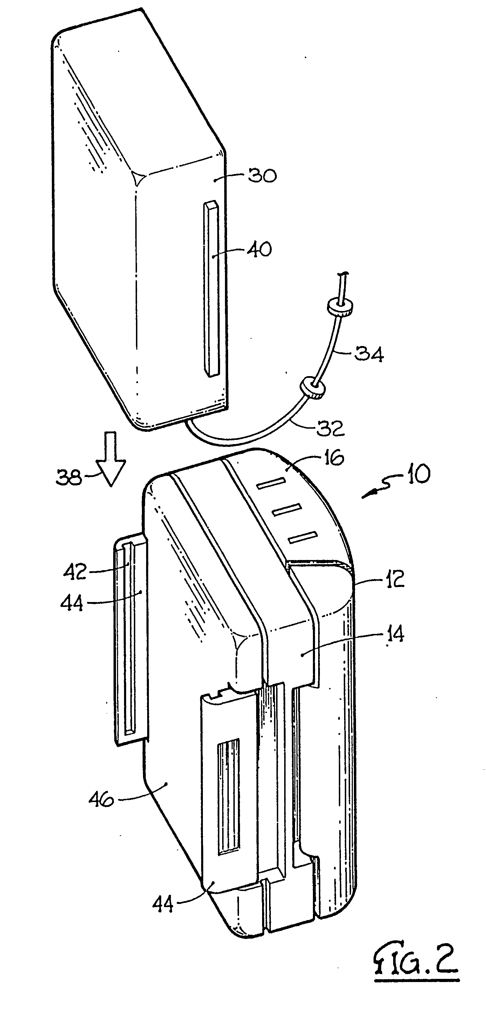

Figure 1 shows the ambulatory programmable pump for dispensing I.V. fluids to a patient in accordance with the present invention which is generally designated 10. The ambulatory programmable pump 10 comprises a foldable casing 12 having a body portion 14, and a cover portion 16. Enclosed in body portion 14 is a computer (not shown) for programmably controlling peristaltic pumping apparatus 36, which apparatus are known to the skilled artisan. Cover portion 16 is hingedly connected to body portion 14 for allowing the cover portion 16 to be moved between an open position as shown in Figure 1, and a closed position, as shown in Figure 2 and discussed further below. In particular, body portion 14 includes a keyboard 18 for inputting data and information to the computer of the ambulatory programmable pump 10. Data input via keyboard 18 by the user of the dispenser is communicated to the user via a display 20 located on the inside 22 of cover portion 16. The information displayed on display 20 also includes step-by-step operating instructions for the user as will be more fully explained below.

The casing 12 has rounded corners 24 to eliminate high impact points should the ambulatory programmable pump 10 be dropped by the user. In addition, the material utilized for casing 12 is high impact resistant material having reliable durability as well as resistance to chemicals. Moreover, pads 26 of keyboard 18 are rubber like for added durability and ease of use in the home environment. In addition, the display 20 includes a transparent shield 28, such as clear plastic, positioned over the display 20 to provide added durability and rugged character to pump 10.

Display 20 is preferably an LCD display of approximate dimensions being preferably from three and one-half (3.5) to four (4) inches wide by five and one-half (5.5) to six and one-half (6.5) inches high, as indicated by the width "w" and height "h" dimensions of Figure 1, respectively. There are large, easy to read characters utilized on the display, with preferably at most ten lines of information displayed at any one time. In addition as will be more fully explained below, data input via keypads depressed on keyboard 18 by the user are held on the display screen 20 until verified as being correct by the user. In addition, the characters displayed on the display screen 20 are the same as, and correspond to, the characters indicated on the individual key pads 26. There is a clear, direct correlation between the key pads 26 and the display 20 to eliminate confusion by the user. Depression of the key pads 26 and their effect on the display screen 20 as well as the general operation of the ambulatory programmable pump 10 is more fully explained below.

Referring now to Figure 2, there is shown the ambulatory programmable pump 10 with the cover 16 in a closed position against body portion 14. A fluid reservoir 20 containing the antibiotics, I.V. fluid, analgesic or other fluid to be infused into the patient, is shown removably detached from the foldable casing 12. In particular, fluid reservoir 30 includes a tube 32 in fluid communication with the reservoir 30 having a pumping section 34 which is inserted in peristaltic pump apparatus 36 as shown in Figure 1, which peristaltic pump apparatus are known by those skilled in the art. The fluid reservoir 30 is removably detachable from foldable casing 12 and is slidably mounted onto the ambulatory programmable pump 10 by sliding the fluid reservoir 30 generally in the direction of arrow 38 so that ridges 40 connected to fluid reservoir 30 slide into tracks 42 formed on the inside of flanges 44 extending from the back 46 of the foldable casing 12.

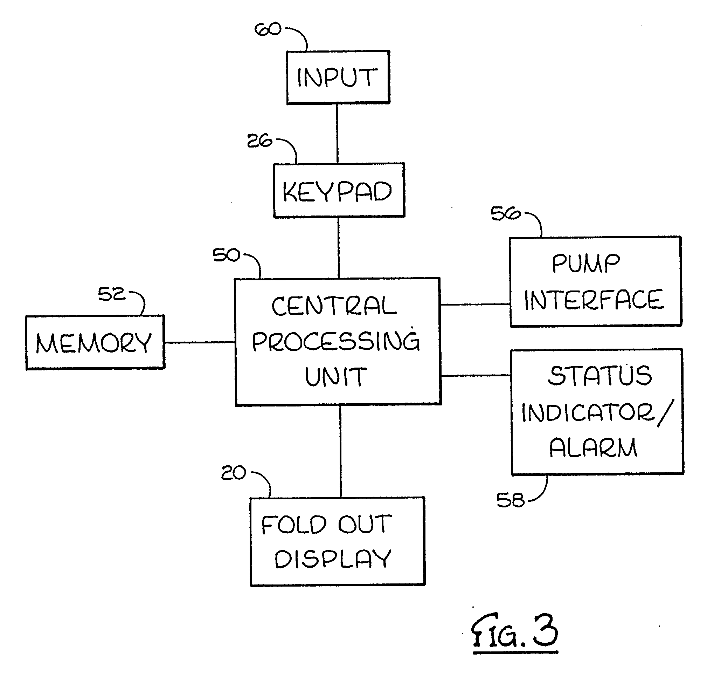

Referring now to Figure 3, there is shown a schematic diagram of the components of the ambulatory programmable pump 10 including a central processing unit 50 connected to the key pad 26 having a memory 52. The central processing unit 50 is housed in the casing 12 and is also connected to the foldout display screen 20. The connection cable (not shown) is routed through hollow hinge connector 54, shown in Figure 1, to protect the integrity of the connection. Also connected to central processing unit 50 is a pump interface 56 for controlling the peristaltic pump mechanism 36, in addition to a status indicator and alarm 58. The user may input 60 information to key pad 26 which transmits information to central processing unit 50 for processing and display on foldout display 20. Various operating parameters of the ambulatory programmable pump 10 are stored in memory 52 and the central processing unit 50 controls the pump interface 56 and the status indicator and alarm 58.

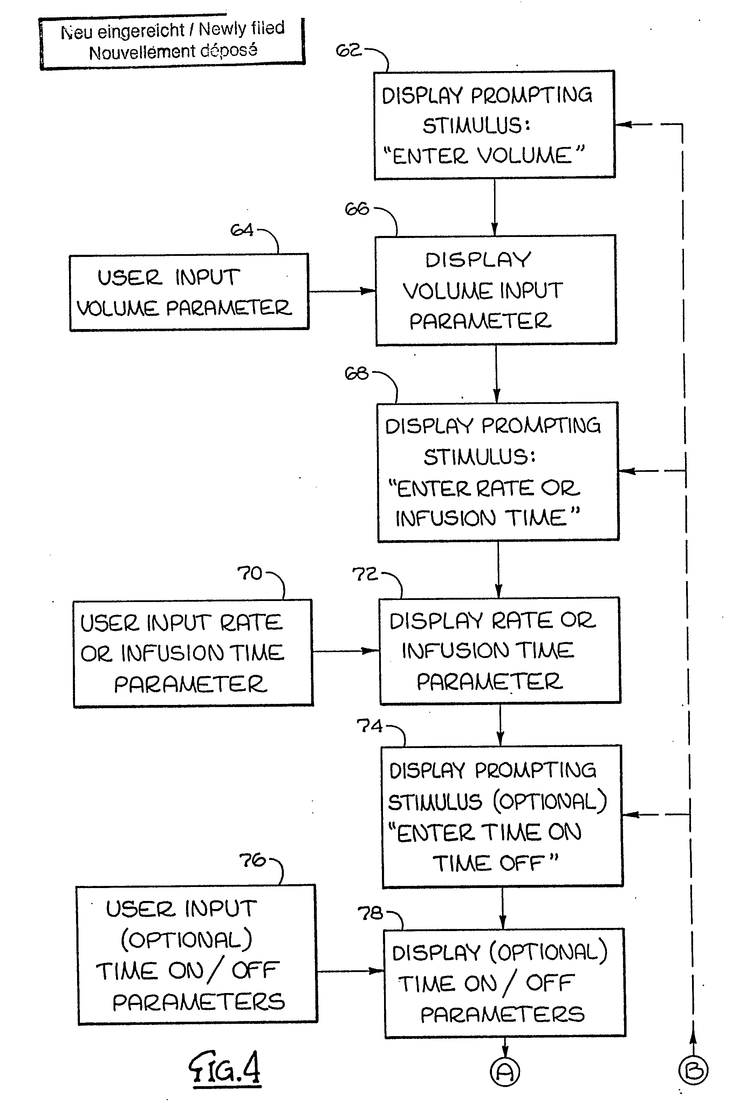

Referring now to Figure 4, there is shown a sequence of prompting stimuli processed by the central processing unit 50 in accordance with the present invention. In particular, after the user has connected the device to the patient and has loaded the fluid reservoir 30 into the ambulatory programmable pump 10, the user inserts the pumping section 34 of tube 32 into the peristaltic pumping apparatus 36 of the ambulatory programmable pump 10. Central processing unit 50 controls display 20. The display 20 has a prompting stimulus to tell the user to "enter volume" 62. Based upon the instructions given by the display 20, the user can then input 64 the volume parameter. Upon input 64 by the user of the volume parameter, the screen displays 66 the volume input parameter. Next, there is a display prompting stimulus 68 "enter rate of infusion time". In response to this being displayed on screen 30 in large letters, the user then can input 70 the rate or infusion time parameter. In a typical application, the user has been instructed by the health care professional as to the proper rate. The rate is typically one to four hundred milliliters per hour in the normal I.V. infusion mode.

In a preferred embodiment, the present device also has a patient controlled analgesic (PCA) mode for administering analgesics or other pain suppressing medications. In the PCA mode, the range of desired rates are from one-tenth (.1) to nineteen and nine-tenths (19.9) milliliters per hour, preferably in one-tenth (.1) increments, or from twenty (20) to one hundred (100) milliliters per hour, in one (1) milliliter increments. The increments of rates, and the numbers for inputting same are conveniently located on key pads 26 of keyboard 18. When the information is input, it is simultaneously displayed on screen 20 to show the user thereof what rate has been input. The user can input either the rate or the infusion time; or if the volume is known and the infusion time is known, the computer 50 can calculate the appropriate rate. Computer 50 will automatically calculate the appropriate parameters based on the instructions given by the health care professional to the user. Thus, once the user inputs the rate or infusion time, it is displayed 72 on the screen 20.

Next, there is an optional display prompting stimulus 74 which displays an easily readable display onto the screen 20 for the user to "enter time on, time off". This requested information is for utilization of intermittent infusion of fluid in the case of intermittent infusion, as compared to normal continuous infusion. Again, this is dependent upon the instructions given by the health care professional as to the appropriate therapy. However,display 20 prompts the user as to exactly which information needs to be utilized by the ambulatory programmable pump 10. In response to the prompting stimulus 74, the use inputs 76 the appropriate "time on, time off" parameters. When such parameters are input 76, the display of "time on, time off" parameters occurs 78 on the screen 20, again indicating to the user clearly and without confusion the data that has been input by the user.

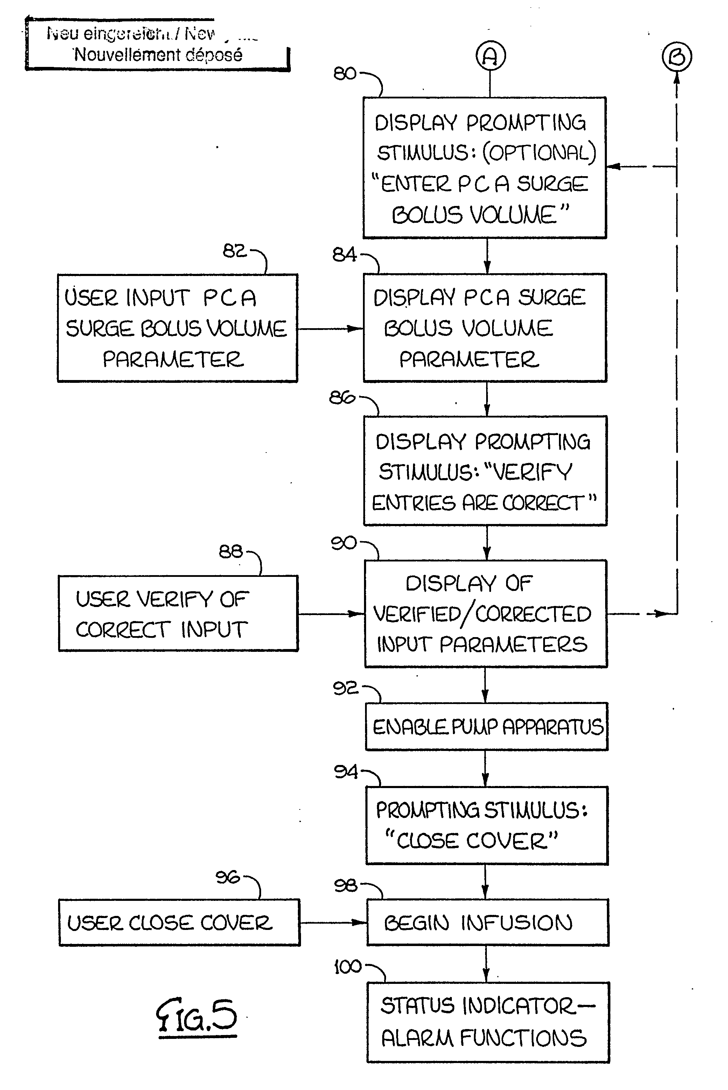

Referring now to Figure 5, there is further shown a continuation of the block diagram of Figure 4 showing the display prompting stimulus in accordance with the present invention. In particular, there is shown that after the display of the "time on, time off" parameters 78 in Figure 4, there is a display prompting stimulus 80 which is optional; "enter PCA surge bolus volume" 80. Based on this prompting stimulus 80, the user selects from the keyboard 18 and inputs the PCA surge bolus volume parameter. This bolus volume is the amount of analgesic or other medicinal fluid which is infused in addition to the constant fluid infusion having been already set by the user of the device. This surge or bolus allows additional volume to be applied intermittently in addition to the constant infusion amount required. The user input 82 causes the display screen 20 to display 84 the PCA surge bolus volume parameter input by the user.

In all of the above situations in which the prompting stimulus causes the user to enter data, the data is preferably displayed on screen 20 in a blinking or other fashion to indicate the data which has been entered, as each number is entered, so that when the total amount or storing of data is correct, the input can then be entered, at which time the data is then displayed in constant or non-blinking fashion on the screen 20. Even after all of the above display prompting stimuli have instructed the user accordingly, there is a display prompting stimulus 86 on the foldout screen 20 which requests the user to "verify entries or correct". The user is then given the opportunity to verify 88 the input and make corrections if required, according to the instructions from the screen 20. In the even data needs to be corrected, the appropriate input is entered into the keyboard 18 and the corrected input is then displayed 90 as verified and corrected input parameters. There is a feedback loop so that the parameter requiring correction is then requested from the user by the appropriate display prompting stimulus 62, 68, 74, or 80 as required and shown in feedback loop B of Figures 4 and 5.

Once the appropriate information and parameters have been input by the user in response to the user prompting stimuli for instructing the user in operation of the pump 10, the user enables the pump apparatus 92 to begin operation of infusing the medications at the rates and in the amounts entered by the user. In addition, in the event optional settings of intermittent infusion are utilized, the time on and time off parameters, as well as the PCA surge bolus volume parameters, are utilized for administering the I.V. fluid to the patient. The ambulatory programmable pump 10 further has prompting stimulus 94 for instructing the user to close the cover 96 to begin infusion 98. During operation of the device, status indicator and alarm functions are continuously monitored 100.

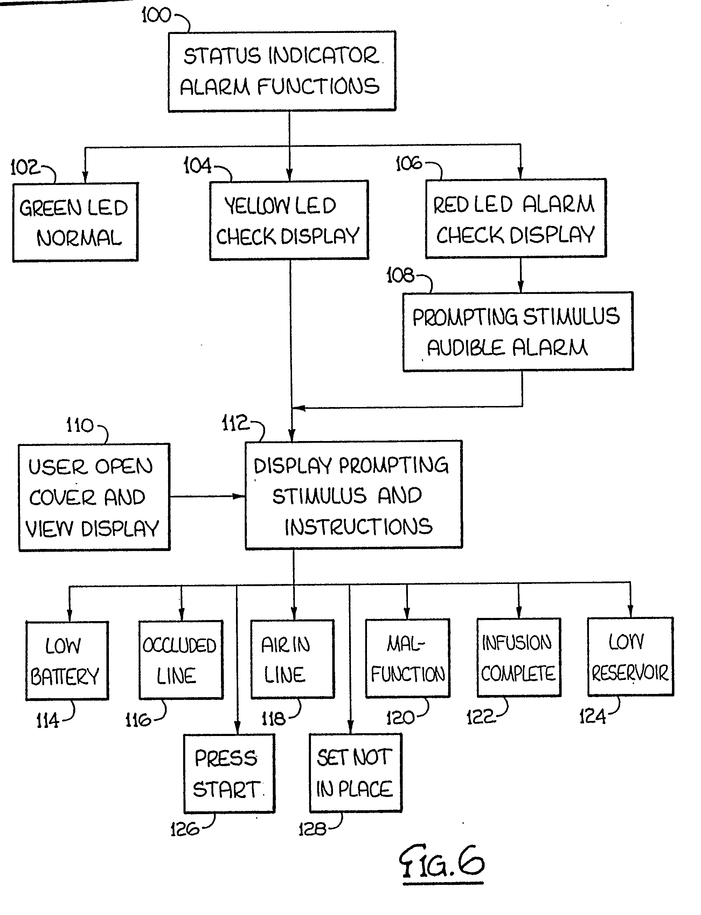

Referring now to Figure 6, there is shown in further detail the block diagram of the prompting stimulus and operation of the ambulatory programmable pump 10 involving the status indicator and alarm functions 100. In particular, connected to the central processing unit 50 and housed in the cover 16 are a green light emitting diode (LED) 102, a yellow LED 104 and red LED display 106. The green LED 102 is a flashing LED which flashes intermittently when operation of the ambulatory programmable pump 10 is in normal operation with no problems indicated. Yellow LED 104 is a flashing LED which indicates that the user should open the cover 16 and check the display 20 for instructions and prompting stimulus accordingly. Yellow flashing LED 104 is an advisory condition. When red LED 106 is flashing it is an alarm condition and the user should check display 20.

The alarm condition also includes a prompting stimulus in the form of an audible alarm 108. The audible alarm 108 has an adjustable volume and adjustable pitch in the range of from five hundred to four thousand Hz, both of which can be adjusted by the user via the key pads 26 to ensure that the user of pump 10 can hear the audible alarm condition in a prompt manner, while allowing adjustment to the particular needs of the user of the pump 10. Accordingly, the user opens 110 the cover and views the display 20.

Display of appropriate stimulus 112 is available for the user in response to the flashing LEDs and alarms and appropriate instructions are given. Depending on the condition, the instructions may include status indicating information on the display screen 20 including low battery 114, occluded line 116, air in line 118, a malfunction in the device 120, infusion complete 122, low fluid reservoir 124, press start button 126, or set not in place 128. In response to each of these conditions, appropriate instructions are also given the user by the display screen 20, according to the particular condition being indicated on the display screen 20.

In addition, the central processing unit memory 52 is designed so that the memory is retained during battery change, and further, the central processing unit 50 is preferably designed so that there is less than five percent (5%) variance in accuracy of the rate and amount of fluid being infused to the patient under normal operating conditions.

While the particular ambulatory programmable pump as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as defined in the appended claims.

## Claims

1. An intravenous fluid infusing device comprising:
a foldable casing;
computer controlled pumping means mounted in said casing for pumping fluid;
a keyboard mounted on said casing for controlling operation of said pumping means; and
display means mounted in said casing and hingedly attached to said keyboard for displaying information in response to input to said keyboard.

2. A device according to Claim 1, wherein:
said foldable casing includes fluid container means for storing fluid to be infused to a patient; and further comprising;
a central processing unit carried in said casing and connected to said pumping means, the operation of said processing unit being controlled by said keyboard; and
said display means being movable between an open position for displaying information to the operator of the pump, said information including step-by-step instructions to operate the pump, and a closed position wherein said display is protected from view.

3. A device according to Claim 2, wherein pressing said keys to enter information causes said information to be displayed on said display.

4. A device according to Claim 3, wherein said entered information is indicated on said display in blinking fashion until verified by the user as correct.

5. A device according to Claim 1 wherein:
said pumping means can pump IV fluid to a patient at desired rates and in desired amounts and said foldable casing comprising a body portion having said keyboard mounted therein; and
said foldable casing comprising a cover portion hingedly attached to said body portion, said cover portion having an inside surface and an outside surface, the inside surface of said cover portion having said display means mounted thereon for displaying information for instructing the user in operation of the dispenser.

6. A device according to Claim 5, wherein said cover portion includes a transparent shield superposed over said display means.

7. A device according to any of Claims 2 to 6, further comprising means for displaying the status of operation of the pump, including the rate of infusion, and the volume of fluid to be infused.

8. A device according to any of Claims 2 to 7, further comprising status indicating lights mounted in said casing.

9. A device according to Claim 8, wherein said status indicating lights comprise flashing light emitting diodes.

10. A device according to any preceding claim, further comprising a fluid reservoir removably attachable to said body portion.

11. A device according to Claim 10, wherein said fluid reservoir is attached to said body portion by a pair of flanges having tracks therein for slidably receiving said fluid reservoir.

12. A device according to any preceding claim, wherein said display means comprises a liquid crystal display.

13. A device according to any preceding claim, further comprising an audible alarm for indicating an alarm condition.

14. A method of operating a fluid transferring device having a peristaltic pumping apparatus, comprising the steps of:

attaching a fluid reservoir in operative engagement to said peristaltic pumping apparatus;

opening a cover carrying a foldable display screen for viewing user instructions generated by said computerized pump;

entering data including transfer rate and volume of said fluid via said keyboard in response to prompting stimulus displayed to the user on said foldable display screen; and

closing said cover and enabling said device to transfer said fluid at the rate and in the amount entered by said user.

FIG.1

FIG.2

FiG.3

62

```
DISPLAY PROMPTING
STIMULUS:
"ENTER VOLUME"
```

66

```
DISPLAY
VOLUME INPUT
PARAMETER
```

64

```
USER INPUT
VOLUME PARAMETER
```

68

```
DISPLAY PROMPTING
STIMULUS:
"ENTER RATE OR
INFUSION TIME"
```

70

```
USER INPUT RATE
OR INFUSION TIME
PARAMETER
```

72

```
DISPLAY RATE OR
INFUSION TIME
PARAMETER
```

74

```
DISPLAY PROMPTING
STIMULUS (OPTIONAL)
"ENTER TIME ON
TIME OFF"
```

76

```
USER INPUT
(OPTIONAL)
TIME ON / OFF
PARAMETERS
```

78

```
DISPLAY (OPTIONAL)
TIME ON / OFF
PARAMETERS
```

Ⓐ          Ⓑ

FIG.4

Ⓐ   Ⓑ

80 — DISPLAY PROMPTING STIMULUS: (OPTIONAL) "ENTER P C A SURGE BOLUS VOLUME"

82 — USER INPUT P C A SURGE BOLUS VOLUME PARAMETER

84 — DISPLAY PCA SURGE BOLUS VOLUME PARAMETER

86 — DISPLAY PROMPTING STIMULUS: "VERIFY ENTRIES ARE CORRECT"

88 — USER VERIFY OF CORRECT INPUT

90 — DISPLAY OF VERIFIED/CORRECTED INPUT PARAMETERS

92 — ENABLE PUMP APPARATUS

94 — PROMPTING STIMULUS: "CLOSE COVER"

96 — USER CLOSE COVER

98 — BEGIN INFUSION

100 — STATUS INDICATOR — ALARM FUNCTIONS

FIG.5

100 —

STATUS INDICATOR
ALARM FUNCTIONS

102 — GREEN LED NORMAL

104 — YELLOW LED CHECK DISPLAY

106 — RED LED ALARM CHECK DISPLAY

108 — PROMPTING STIMULUS AUDIBLE ALARM

110 — USER OPEN COVER AND VIEW DISPLAY

112 — DISPLAY PROMPTING STIMULUS AND INSTRUCTIONS

LOW BATTERY — 114

OCCLUDED LINE — 116

AIR IN LINE — 118

MAL-FUNCTION — 120

INFUSION COMPLETE — 122

LOW RESERVOIR — 124

PRESS START — 126

SET NOT IN PLACE — 128

FIG.6

European Patent
Office

**EUROPEAN SEARCH REPORT**

Application Number

EP 89 30 7677

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-8 500 523  (BAXTER TRAVENOL)  --- | | A 61 M   5/142 |
| A | WO-A-8 400 691  (AMERICAN HOSPITAL SUPPLY CORP.)  --- | | |
| A | AUTUMN, no. 157, 1986, pages 26-28, Tokyo, JP; G. YAMAZAKI et al.: "PC-Compatible Laptop computer"  ----- | | |

TECHNICAL FIELDS
SEARCHED (Int. Cl.5)

A 61 M
G 06 F

The present search report has been drawn up for all claims

| Place of search | Date of completion of the search | Examiner |
|---|---|---|
| THE HAGUE | 23-01-1990 | CLARKSON P.M. |